# EUROPEAN PATENT APPLICATION

(11) **EP 1 666 593 A1**
(43) Date of publication of application: **07.06.2006**
(21) Application number: 04771783.0
(22) Date of filing: 18.08.2004
(51) Int. Cl.: C12N 15/11

(54) **IMPROVED siRNA MOLECULE AND METHOD OF INHIBITING GENE EXPRESSION WITH THE USE OF THE SAME**

(30) Priority: 18.08.2003 JP 2003294504; 24.12.2003 JP 2003427970
(71) Applicant: Japan Health Science Foundation, Tokyo 103-0001 (JP)
(72) Inventor: HOHJOH, Hirohiko, Tokyo 1870031 (JP)
(74) Representative: Ahner, Francis
(86) International application number: PCT/JP2004/011822
(87) International publication number: WO 2005/017154

(57) **Abstract**

The present invention relates to a double-stranded RNA molecule improved to control the gene expression suppressing effect of an siRNA. The double-stranded RNA molecule of the present invention is designed such that, in a double-stranded RNA molecule capable of suppressing the expression of a target gene in a cell by RNAi, one or more nucleotides in order from the 3'- or 5'-end of the sense strand of double-stranded part in said RNA molecule are not complementary to the antisense strand. Further, in the double-stranded RNA molecule of the present invention, the sense strand of the double-stranded part has adequate number of nucleotides which are complementary to the antisense strand for enabling the hybridization of both strands in the cell.

## Description

### BACKGROUND OF THE INVENTION

### Field of the Invention

The present invention relates to gene silencing by the RNAi phenomenon, and more specifically to improved siRNAs and a method for suppressing the expression of a target gene using this phenomenon.

### Background Art

RNAi (RNA interference) is a mechanism of a sequence-specific posttranscriptional gene suppression triggered by double-stranded RNAs (dsRNAs). This phenomenon has been found in various kinds of species including flies, insects, protozoa, vertebrates, and higher plants. A majority of studies on the molecular mechanism underlying RNAI activity has been conducted using *Drosophila* and *Caenorhabditis* elegans and demonstrated that RNA fragments of 21-25 nucleotides, referred to as siRNAs (short interfering RNAs) are essential sequence-specific mediators of RNAi (Hammond, S.M. et al., Nature 404, 293-296, 2000; Parrish, S. et al., Mol. Cell. 6, 1077-1087, 2000; Zamore, P.D. et al., Cell 101, 25-33, 2000) and that siRNAs are generated from long double-stranded RNAs by digestion with an RNase III-like nuclease, Dicer (Brenstein, E. et al., Nature 409, 363-366, 2001; Elbashir, S.M. et al., Genes Dev. 15, 188-200, 2001).

In mammalians, it was initially thought that RNAi occurs only in oocytes and preimplantation embryos. Mammalian cells, in general, possess a rapid and non-specific RNA degradation pathway involving the sequence-non-specific RNase, RNase L, and a rapid translation inhibition pathway involving the interferon-inducible, dsRNA-activated protein kinase (PKR), both of which can be activated by double-stranded RNAs of 30 nucleotides or more. Thus, the abovementioned responses to double-stranded RNAs of 30 nucleotides or more may mask the sequence-specific RNAi activity in mammalian cells except for undifferentiated cells as well as differentiated cells that lack PKR. Recently, it has been reported that synthetic 21-nucleotide siRNA duplexes can specifically inhibit the expression of endogenous genes in cultured mammalian cells (Elbashir, S.M. et al., Nature 411, 494-498, 2001).

The principle of the suppression of gene expression by RNAi is thought to be as follows. First, when introduced in a cell, siRNA is incorporated into a multi-protein complex to form an RISC (RNA-induced silencing complex). Then, this RISC bonds to mRNA from a target gene and its nuclease activity cleaves the site to which siRNA is attached in the mRNA. As a result, the expression of the protein by the target gene is inhibited.

In recent years, a method of using the RNAi phenomenon by the introduction of a synthetic siRNA into a cell has drawn attention and been utilized as a method for the suppression of gene expression. Further, the relation of the structure of siRNA and its effect on the suppression of gene expression has also been studied.

For example, in a report by Hohjoh H. (Hohjoh H., FEBS Letters 521, 195-199, 2002), various types of synthetic oligonucleotide duplexes designed for the *Photinus* luciferase gene were tested on their effect on suppressing the expression of said gene in mammalian cells. This report describes that 2-ribonucleotide overhangs at each 3'-end of sense and antisense strands are inter alia preferable, that the structure of antisense strands is important, and that the resultant effect on suppression of gene expression varies dependent on target sequences of the gene of interest.

In a report by Hamada M. et al (Hamada M. et al., Antisense Nucleic Acid Drug Dev. 12, 301-309, 2002), the effect on suppressing the expression of a target gene is studied using siRNAs into which one or more discontinued mismatches with a target sequence are introduced at sites other than each 3'-end of sense and/or antisense strands. This report describes that the effect on suppressing the gene expression is reduced by the mismatches with the target sequence in the antisense strand while the mismatches in the sense strand have no effect on suppressing the gene expression.

### SUMMARY OF THE INVENTION

The present inventor has found that in siRNA, the effect of siRNA on suppressing the gene expression is enhanced by introducing mismatches with the antisense strand in several nucleotides at the 3'-end of the sense strand in the double-stranded part. Further, the present inventor has found that in siRNA, the effect of siRNA on suppressing the gene expression is reduced by introducing mismatches with the antisense strand in several nucleotides at the 5'-end of the sense strand in the double-stranded part. The present invention is based on these findings.

Accordingly, an object of the present invention is to provide a double-stranded RNA molecule which is an siRNA so improved to control its effect on suppressing the gene expression, and a method for suppressing the gene expression using this RNA molecule.

In a first aspect of the present invention, there is provided a double-stranded RNA molecule capable of suppressing the expression of a target gene in a cell by RNAi, which is designed such that one or more nucleotides in order from the 3'-end of the sense strand of double-stranded part in said RNA molecule are not complementary to the antisense strand, wherein the sense strand of the double-stranded part has adequate number of nucleotides which are complementary to the antisense strand for enabling the hybridization of both strands in said cell.

In a second aspect of the present invention, there is provided a double-stranded RNA molecule capable of suppressing the expression of a target gene in a cell by RNAi, which is designed such that one or more nucleotides in order from the 5'-end of the sense strand of double-stranded part in said RNA molecule are not complementary to the antisense strand, wherein the sense strand of the double-stranded part has adequate number of nucleotides which are complementary to the antisense strand for enabling the hybridization of both strands in said cell.

Further, a method for suppressing the gene expression of the present invention is a method for suppressing the expression of a target gene in a cell, comprising a step of introducing a double-stranded RNA molecule of the present invention into the cell.

According the present invention, it becomes possible to control the efficiency of the suppression of the expression in the method for the suppression of the expression of the target gene using an RNAi phenomenon.

### BRIEF DESCRIPTION OF THE DRAWINGS

Fig. 1 shows graphs illustrating the results of the dual luciferase assay showing the effect of various types of siRNAs on suppressing the expression of the *Photinus* luciferase gene in HeLa cells.
Fig. 2 shows graphs illustrating the effect of various types of siRNAs on suppressing the expression of endogenous genes in HeLa cells.
Fig. 3 shows bar graphs illustrating the level of expression of reporters from two types of plasmids, using the siRNA molecule which contains mismatches at the 3'-end of the sense strand (La21-3'm2) and the siRNA molecule without mismatch (La21-conv.).

### DETAILED DESCRIPTION OF THE INVENTION

The term "double-stranded RNA" as used herein means an RNA molecule which is obtained by overall or partial hybridization of two single-stranded RNA molecules, The number of nucleotides in each of the single-stranded RNAs can be different one another. Further, either one or both of the nucleotide strands in the double-stranded RNA may contain a single-stranded part (overhang).

The term "double-stranded part" as used herein means a part where in a double-stranded RNA, nucleotides of both strands are paired, namely a part excluding a single-stranded part in the double-stranded RNA.

The term "sense strand" as used herein means a nucleotide strand that has a sequence homologous to a coding strand of a gene, and the term "antisense strand" means a nucleotide strand that has a sequence complementary to the coding strand of the gene.

The term "complementary" as used herein means that two nucleotides can be paired under hybridization conditions, for example, relations between adenine (A) and thymine (T) or uracil (U), and between cytosine (C) and guanine (G).

A double-stranded RNA molecule capable of suppressing the expression of a target gene in a cell by RNAi can be designed easily by those skilled in the art based on the existing knowledge on RNAi. In general, first, a region specific to the target gene is selected and a ribonucleotide strand which is specifically hybridizable with this region in a cell is to be the antisense strand of the abovementioned double-stranded RNA molecule. The term "specific region" as used herein means a region having a sequence that cannot found in other nucleic acid molecules in the cell in which the suppression of the expression of the target gene is to be carried out. Further, the term "specifically hybridizable" means that the antisense strand does not hybridize with any nucleic acid molecules other than the target gene and its transcription products in the cell in which the suppression of the expression of the target gene is to be carried out. The abovementioned antisense strand is to contain a sequence corresponding to the abovementioned specific region; however, it may not necessarily have a sequence completely complementary to the abovementioned region and may contain noncomplementary nucleotides as long as it is specifically hybridizable with the specific region. However, the abovementioned antisense strand is preferably to have a sequence completely complementary to the abovementioned region. The sense strand of the double-stranded RNA molecule is to contain a sequence completely complementary to the antisense strand.

The abovementioned specific region to be selected in the target gene is selected preferably only from exons in the target gene. Further, it is generally believed to be preferable to select the abovementioned specific region from portions excluding 5'- and 3'-nontranslated regions (UTRs) and the proximity to the translation initiation codon; for example, in a sequence containing both exons and introns, it is a region preferably 50 or more nucleotides distant from the 3'-end of the translation initiation codon, a region more preferably 75 or more nucleotides distant, most preferably 100 or more nucleotides distant, from the 3'-end of the translation initiation codon. The length of the abovementioned specific region is not particularly limited but is preferably 15 or more nucleotides long, more preferably 17 or more nucleotides long, still more preferably 19 or more nucleotides long, further more preferably 19-23 nucleotides long, and most preferably 19-21 nucleotides long.

The antisense strand of the abovementioned RNA molecule may have a single-stranded part (overhang) at the 3'-end of the double-stranded part paired with the nucleotides of the sense strand. The nudeotide sequence of this single-stranded part can be either a sequence related or unrelated to the target gene and its length is not particularly limited but is preferably a sequence of two or more nucleotides and more preferably two uracil residues (UU). The sense strand can also have a similar overhang.

The double-stranded RNA molecule according to a first aspect of the present invention is designed such that, in the abovementioned double-stranded RNA molecule, one or more nucleotides in order from the 3'-end of the sense strand of double-stranded part in said RNA molecule are not complementary to the antisense strand. Namely, the sense strand of the double-stranded RNA molecule according to the first aspect possesses one or more mismatches with the antisense strand in order from the 3'-end of double-stranded part of the sense strand. Thereby the effect on the suppression of the expression of the target gene is enhanced. However, these sense strand and antisense strand have to be maintained as a double-stranded structure in the cell and thus the number of the mismatched nucleotides is restricted by the number of nucleotides which are complementary to the antisense strand necessary to maintain the double-stranded structure. Therefore, in the double-stranded RNA molecule according to the first aspect of the present invention, the sense strand of the double-stranded part has adequate number of nucleotides which are complementary to the antisense strand for enabling the hybridization of both strands in the cell.

Whether or not two ribonucleotide sequences hybridize in a cell can be examined by those skilled in the art. For example, hybridization conditions in a cell of interest can be reproduced in vitro and the two ribonucleotide strands can be added thereto to examine whether or not they hybridize.

In a preferred embodiment for the double-stranded RNA molecule according to the first aspect of the present invention, the number of the nucleotides which are not complementary to the antisense strand in order from the 3'-end of the sense strand of the double-stranded part is preferably 1 to 4, more preferably 2.

In order to efficiently suppress the expression of the target gene in the cell, in addition to the abovementioned mismatch at the 3'-end of the sense strand, it is advantageous to introduce a mismatch in one nucleotide located at position 11-13 from its 3'-end. Therefore, in a preferred embodiment of the present invention, the double-stranded RNA molecule according to the first aspect of the present invention is designed such that one additional nucleotide located at position 11-13, more preferably at position 12, from the 3'-end of the sense strand of the double-stranded part is not complementary to the antisense strand.

It has been confirmed by the experimental data using a double-stranded RNA molecule in which mismatches are introduced in two nucleotides at the 3'-end and in one nucleotide at position 12 from the 3'-end in a 19-20 nucleotide long sense strand, that the double-stranded RNA molecule having a mismatch in one nucleotide located at position 11-13 from the 3'-end of the sense strand of the double-stranded part is advantageous in enhancing the effect on suppressing the gene expression. Further, the site of this mismatch is close to the cleavage site of the target gene transcription product by RISC. Therefore, in the double-stranded RNA molecule according to the first aspect of the present invention, in addition to the mismatches at the 3'-end of the double-stranded part of the sense strand, a mismatch may be introduced in one nucleotide located at nucleotide position 1-3 in 5'- or 3'-direction from a site on the sense strand of the double-stranded part, the site corresponding to the cleavage site of the target gene transcription product by RISC. The cleavage site of the target gene transcription product by RISC can be determined by those skilled in the art according to the sequence in the specific region of the target gene contained in the double-stranded RNA molecule; however, it is typically in the central part of the sequence of the abovementioned specific region.

In a preferred embodiment for the double-stranded RNA molecule according to the first aspect of the present invention, in addition to the mismatches at the 3'-end of the double-stranded part of the sense strand, a mismatch is introduced in one nucleotide located at nucleotide position 1-3, preferably at nucleotide position 2, in 5'-direction from the nucleotide in the center when the double-stranded part of the sense strand has an odd number of nucleotides, while it is introduced in one nucleotide located at nucleotide position 1-3, preferably at nucleotide position 2, in 5'-direction from the nucleotide at the 3'-side of the center when the double-stranded part of the sense strand has an even number of nucleotides.

The double-stranded RNA molecule according to a second aspect of the present invention is designed such that, in the double-stranded RNA molecule capable of suppressing the expression of the target gene in the cell by RNAi, one or more nucleotides in order from the 5-end of the sense strand of double-stranded part in said RNA molecule are not complementary to the antisense strand. Namely, the sense strand of the double-stranded RNA molecule according to the second aspect possesses one or more mismatches with the antisense strand in order from the 5'-end of double-stranded part of the sense strand. Thereby the effect on the suppression of the expression of the target gene is reduced. However, these sense strand and antisense strand have to be maintained as a double-stranded structure in the cell and thus the number of the mismatched nucleotides is restricted by the number of nucleotides which are complementary to the antisense strand necessary to maintain the double-stranded structure. Therefore, in the double-stranded RNA molecule according to the second aspect of the present invention, the sense strand of the double-stranded part has adequate number of nucleotides which are complementary to the antisense strand for enabling the hybridization of both strands in the cell.

In a preferred embodiment for the double-stranded RNA molecule according to the second aspect of the present invention, the number of the nucleotides which are not complementary to the antisense strand in order from the 5'-end of the sense strand of the double-stranded part is preferably 1 to 4, more preferably 2.

The double-stranded RNA molecule according to the second aspect of the present invention can further contain a part or all of the mismatches in the sense strand as described above with regard to the double-stranded RNA molecule according to the first aspect. Thereby the effect on the suppression of the expression of the target gene can be finely controlled.

It is known that when a long double-stranded RNA molecule is introduced into a mammalian cell, double-stranded RNA-dependent protein kinase and 2',5'-oligoadenylate synthetase are induced, which may results in cell death. Therefore, the double-stranded RNA molecule of the present invention preferably does not induce the double-stranded RNA-dependent protein kinase or 2' ,5'-oligoadenylate synthetase in mammalian cells. The strand length of the double-stranded RNA molecule which satisfies this condition is easily understood by those skilled in the art. It is known that the abovementioned cell death is generally caused by 30 or more nucleotide-long double-stranded RNA molecules; therefore, the double-stranded RNA molecule of the present invention has a strand length of preferably 29 or less nucleotides, more preferably 25 or less nucleotides. The term "strand length" as used herein means not only a nucleotide length of the double-stranded part of the double-stranded RNA molecule but also includes that of the single-stranded part.

The double-stranded RNA molecule of the present invention is capable of controlling the effect on suppressing the gene expression by RNAi, which is supported by the experimental data described in this specification. According to these experimental data, it is demonstrated that in siRNAs, the effect on suppressing the gene expression by RNAi is enhanced when mismatches are introduced at the 3'-end of the sense strand, whereas the effect is reduced when mismatches are introduced at the 5'-end thereof. Such control of the effect on suppressing the gene expression is conferred by controlling the orientation of the incorporated siRNA in the RNA-induced silencing complex (RISC). Namely, when mismatches introduced into one end of an siRNA unwind the hybridization at said end, the siRNA is incorporated into RISC from this end; then of the two strands included in the siRNA, the strand incorporated from the 5'-end functions as an RNAi mediator (true antisense strand). Therefore, in an siRNA designed for controlling the target gene expression, the effect on suppressing the expression of the target gene can be enhanced by modifying said siRNA to be incorporated into RISC from the 5'-end of its antisense strand; on the other hand, the effect on suppressing the expression of the target gene can be reduced by modifying said siRNA to be incorporated into RISC from the 5'-end of its sense strand.

Thus, according to the present invention, there is provided a double-stranded RNA molecule capable of suppressing the expression of a target gene in a cell by RNAi, which is modified such that said double-stranded RNA molecule is incorporated into an RNA-induced silencing complex from the side of 5'-end of the antisense strand. An embodiment of such modification is described above with regard to the double-stranded RNA molecule according to the first aspect of the present invention. By such modification, the effect on suppressing the expression of the target gene is enhanced.

Further, according to the present invention, there is provided a double-stranded RNA molecule capable of suppressing the expression of a target gene in a cell by RNAi, which is modified such that said double-stranded RNA molecule is incorporated into an RNA-induced silencing complex from the side of 5'-end of the sense strand. An embodiment of such modification is described above with regard to the double-stranded RNA molecule according to the second aspect of the present invention. By such modification, the effect on suppressing the expression of the target gene is reduced.

The double-stranded RNA molecule of the present invention can suppress the expression of a target gene in a cell. Thus, according to the present invention, there is provided a method for suppressing the expression of a target gene in a cell, comprising a step of introducing the double-stranded RNA molecule of the present invention into the cell.

The cell can be any cell which can trigger the RNAi phenomenon by siRNAs, including cells derived from insects, worms, plants, mammals, and the like, preferably mammalian cells. Examples of the cells derived from plants include those derived from tomatoes, nicotiana, *Arabidopsis thaliana,* and rice plants. Examples of cells derived from mammals include those derived from mice, hamsters, and humans. Further, examples of suitable cultured cells include HeLa cells, NIH/3T3 cells, COS-7 cells, and 293 cells.

A method for introducing the double-stranded RNA molecule of the present invention into the cell is not particularly limited and may be any method which can introduce the double-stranded nucleic acid molecule into the cell. As for the method of the present invention, a particularly suitable method for introducing the double-stranded RNA molecule into the cell is a transfection method using liposomes, preferably cationic liposomes, which can be easily carried out using a commercial transfection reagent such as Lipofectamine 2000 transfection reagent (Invitrogen), Oligofectamine transfection reagent (Invitrogen), jetSI transfection reagent (Polyplus-transfection), and TransMessenger (Qiagen).

Alternatively, the suppression of the expression of a target gene utilizing the double-stranded RNA molecule of the present invention can be carried out by introducing into the cell of interest a vector which expresses the double-stranded RNA molecule of the present invention in a cell. In this method, either two types of vectors which can individually express the sense strand and antisense strand of the double-stranded RNA molecule of the present invention, or one vector which contains both of a DNA encoding the sense strand of the double-stranded RNA molecule of the present invention and a DNA encoding its antisense strand can be used. Thus, according to another embodiment of the present invention, there is provided a method for suppressing the expression of a target gene in a cell, comprising a step of introducing a combination of a vector containing a DNA encoding the sense strand of the double-stranded RNA molecule of the present invention and a vector containing a DNA encoding the antisense strand of said RNA molecule, or a vector comprising both of a DNA encoding the sense strand of the double-stranded RNA molecule of the present invention and a DNA encoding the antisense strand of said RNA molecule, into the cell.

The abovementioned vector can be any one which is capable of expressing one or both of the strands of the double-stranded RNA molecule in a cell. Accordingly, the abovementioned vector contains DNA(s) encoding the strand(s) of the double-stranded RNA molecule such that the DNA(s) can be transcribed in the cell. Such vector can be easily constructed by those skilled in the art; for example, elements such as promoters and terminators can be ligated in a functional form, if necessary. Any promoter which can function in the cell of interest can be used; for example, either constitutive promoters or inducible promoters can be used. Further, the same promoter as that controlling the expression of the target gene can be used; thereby, the double-stranded RNA molecule of the present invention to decompose the transcription product of the target gene can be produced at the same time when said transcription product is produced. The introduction of the constructed vector into the cell of interest can be appropriately carried out by those skilled in the art by a method known in the art.

In particular, it is preferable to use a single vector capable of expressing both strands of the double-stranded RNA molecule of the present invention. Such a vector can be one that expresses the two strands of the double-stranded RNA molecule separately or one that expresses the double-stranded RNA molecule as a hairpin double-stranded RNA in which the two strands are linked by a linker sequence. A vector that expresses the double-stranded RNA molecule of the present invention as a hairpin double-stranded RNA can be appropriately constructed by those skilled in the art; for example, it can be constructed according to the descriptions in literature (Bass, B.L., Cell 101, 235-238, 2000; Tavernarakis, N. et al., Nat. Genet. 24, 180-183, 2000; Malagon, F. et al., Mol. Gen. Genet. 259, 639-644, 1998; Parrish, S. et al., Mol. Cell 6, 1077-1087, 2000).

The double-stranded RNA molecule of the present invention and the method for suppressing the gene expression utilizing said RNA molecule can target a variety of genes; for example, a gene for which an analysis of its function in a cell is desired can be targeted. Further, by targeting cancer genes, virus genes and the like and suppressing the expression of these genes, their functions can be elucidated, and furthermore by suppressing the expression of these genes in cells existing in the body, treatment of diseases and disorders can be enabled.

### EXAMPLE

### Example 1: Suppression of the expression of Photinus luciferase gene by various siRNAs in HeLa cells

Conventional siRNAs and siRNAs each carrying introduced mismatches with its antisense strand in various sites of its sense strand, against the *Photinus* luciferase gene, were designed to examine their effect on the suppression of the expression of the *Photinus* luciferase gene.

The nucleotide sequences of the various siRNAs thus designed are shown in Table 1 below. In Table 1, for each siRNA, the sense strand and antisense strand are aligned on the top row and the bottom row, respectively, and nucleotides complementary to each other are shown with asterisks "*" between the strands. Further, as for the name of each siRNA, "La2" and "La21" represent the target sequences in the *Photinus* luciferase gene; according to the numbering system in the expression plasmid pGL3-control (Promega) used for the introduction of said gene into the cell, "La2" targets the nucleotides at position 282-300 and "La21" targets the nucleotides at position 340-358. Further, "conv." represents an siRNA which is designed based on the conventional design standard and thus contains no mismatch. Furthermore, "3'BL" represents that the siRNA contains no protrusion (overhang) at the 3'-end of the sense strand.

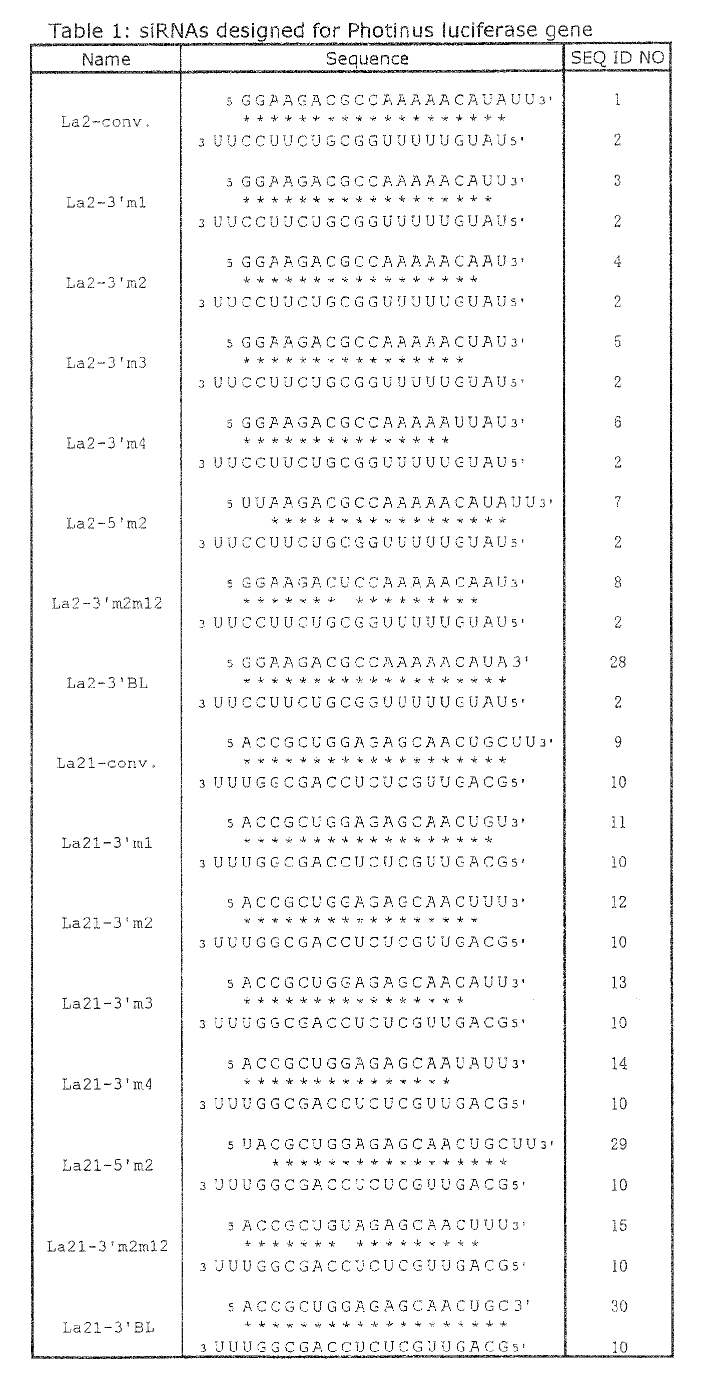

The abovementioned paired oligoribonucleotides were synthesized and 20 µM each of them were each mixed in an annealing buffer (30 mM HEPES pH 7.0, 100 mM potassium acetate, and 10 mM magnesium acetate), heat-denatured at 90°C for 3 minutes, and then annealed at 37°C for 24 hours. In this way, the individual siRNAs were obtained.

HeLa cells were grown at 37°C in Dulbecco's modified Eagle's medium (Sigma) supplemented with 10% fetal bovine serum (Life Technologies), 100 U/ml penicillin (Life Technologies) and 100 µg/ml streptomycin (Life Technologies) in a 5% CO₂ atmosphere.

The day before transfection, the HeLa cells grown were trypsinized, diluted with the fresh medium without antibiotics and seeded into 24-well culture plates (appropriately 0.5 × 10⁵ cell/well in 0.5 ml of the culture medium). Next, the culture medium was replaced with 0.5 ml of OPTI-MEMI (Life technologies), and to each well, 0.25 µg of pGL3-control plasmid (Promega) to *express Photinus* luciferase, 0.05 µg of phRL-TK plasmid (Promega) to express *Renilla* luciferase as a control, and 0.24 µg of individual siRNA were applied. Cotransfection of the two kinds of reporter plasmids and the individual siRNA was carried out using a Lipofectamine 2000 transfection reagent (Invistrogen).

Then, the cells were incubated at 37°C for 4 hours, and after adding 0.5 m! of the fresh culture medium without antibiotics, further incubated at 37°C. Cell lysate was prepared 24 hours after transfection and subjected to luciferase expression assay using Dual-Luciferase Reporter Assay System (Promega).

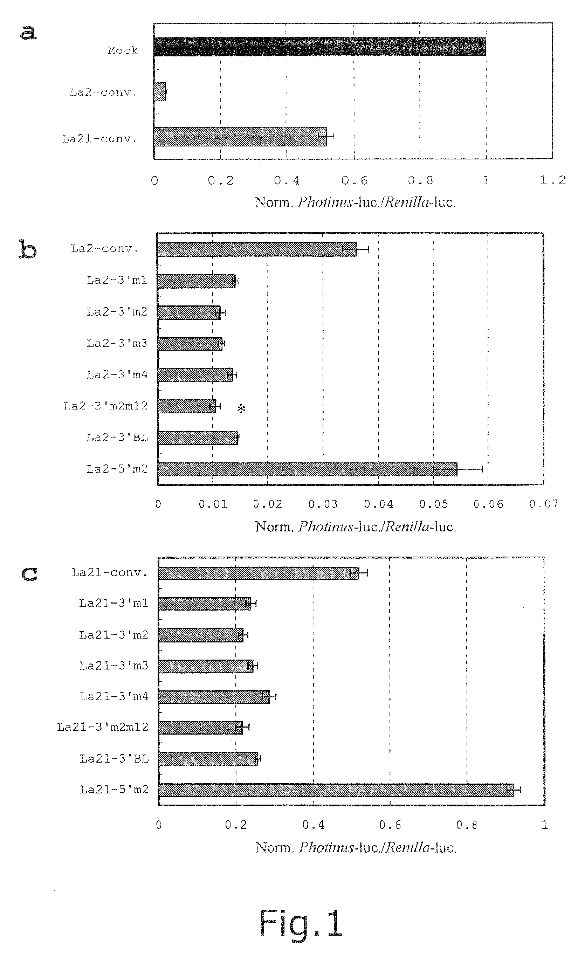

Fig. 1 shows the results of the dual luciferase assay. In Fig. 1, ratios of target *(Photinus)* luciferase activity to control *(Renilla)* luciferase activity when individual siRNAs were used are shown. The ratios of these two kinds of luciferase activities are normalized to the ratio obtained for a control sample using a double-stranded RNA (Mock, Qiagen) which triggers no gene expression suppression, setting the ratio to be 1.0. Data are averages of at least four independent experiments and error bars on the graph represent standard errors.

Fig. 1a shows that La2-conv. exhibited gene expression suppression as strongly as about 98% while La21-conv. exhibited gene expression suppression as moderately as about 50%. This difference in the gene expression-suppressing effect between La2-conv. and La21-conv. is thought to be due to the difference in the target sequence in the *Photinus* luciferase gene.

Fig. 1b shows the gene expression-suppressing effect by various types of siRNAs which target La2 in the *Photinus* luciferase gene. Comparisons La2-conv. versus La2-3'ml, La2-3'm2, La2-3"m3, and La2-3'm4 reveal that the introduction of 1-4 nucleotide mismatches in the 3'-end of the siRNA sense strand increased the target gene expression-suppressing effect by 2- to 3-fold. Among them, the siRNA carrying two nucleotide mismatches in the 3'-end of the sense strand demonstrated the highest gene expression-suppressing effect. Further, the siRNA (La2-3'm2m12) carrying a mismatch in the nucleotide at position 12 from the 3'-end of the sense strand in addition to the two nucleotide mismatches at the 3'-end of the sense strand demonstrated a yet higher level of gene expression-suppressing effect. In contrast, the siRNA (La2-5'm2) carrying two nucleotide mismatches at the 5'-end of the sense strand showed a lower gene expression-suppressing effect than the conventional siRNA (La2-conv.), which demonstrates that the introduction of mismatches at the 5'-end of the siRNA sense strand reduces the effect of the siRNA on the gene expression suppression.

Fig. 1c shows the gene expression-suppressing effect by various types of siRNAs which target La21 in the *Photinus* luciferase gene. Comparisons La21-conv. versus La21-3'ml, La21-3'm2, La21-3'm3, and La21-3'm4 reveal that the introduction of 1-4 nucleotide mismatches in the 3'-end of the siRNA sense strand increased the target gene expression-suppressing effect by 2- to 3-fold. Among them, the siRNA carrying two nucleotide mismatches in the 3'-end of the sense strand demonstrated the highest gene expression-suppressing effect. Further, the siRNA (La21-3'm2m12) carrying a mismatch in the nucleotide at position 12 from the 3'-end of the sense strand in addition to the two nucleotide mismatches in the 3'-end of the sense strand demonstrated a yet higher level of gene expression-suppressing effect. In contrast, the siRNA (La21-5'm2) carrying two nucleotide mismatches in the 5'-end of the sense strand showed a lower gene expression-suppressing effect than the conventional siRNA (La21-conv.), which demonstrates that the introduction of mismatches at the 5'-end of the siRNA sense strand reduces the effect of the siRNA on the gene expression suppression. These results agree with the results obtained by using various siRNAs targeting La2 as above; therefore, it is understood that the augmentation of the gene expression-suppressing effect by the introduction of mismatches in the 3'-end of the siRNA sense strand as well as the introduction of a mismatch in the nucleotide at position 12 from the 3'-end is not controlled by the position of the target sequence in the target gene, the nucleotide sequence of the target sequence, the gene expression-suppressing efficiency by conventional siRNAs without mismatches designed against the target sequence, or the like.

### Example 2: Suppression of the expression of Lamin A/C gene and Dnmt1 gene in HeLa cells by various siRNAs

As to the endogenous Lamin A/C gene and Dnmt1 gene capable of being expressed in HeLa cells, conventional siRNAs and siRNAs each carrying introduced mismatches with its antisense strand in various sites of its sense strand, directed against these genes, were designed to examine their effect on the suppression of the expression of the abovementioned genes.

The nucleotide sequences of the various siRNAs thus designed are shown in Table 2 below. In Table 2, for each siRNA, the sense strand and antisense strand are aligned on the top row and the bottom row, respectively, and nucleotides complementary to each other are shown with asterisks "*" between the strands. Further, as for the name of each siRNA "Lam" and "Dn1" represent the genes to be targeted, namely the Lamin A/C gene and Dnmt1 gene. According to the numbering system in which the translation initiation codon "A" in the mRNA sequence of each gene is set to be 1, "Lam" targets the nucleotides at position 829-851 in mRNA from the Lamin A/C gene, "Dn1(#1)" targets the nucleotides at position 70-89 in mRNA from the Dnmt1 gene, and "Dn1(#2)" targets the nucleotides at position 185-203 in mRNA from the Dnmt1 gene. Further, "Nat.Lam" is an siRNA which targets the Lamin A/C gene and its target sequence is described in literature (Elbrashir, S.M. et al., Nature 411; 494-498, 2001). Further, "conv." represents an siRNA which is designed based on the conventional design standard and thus contains no mismatch.

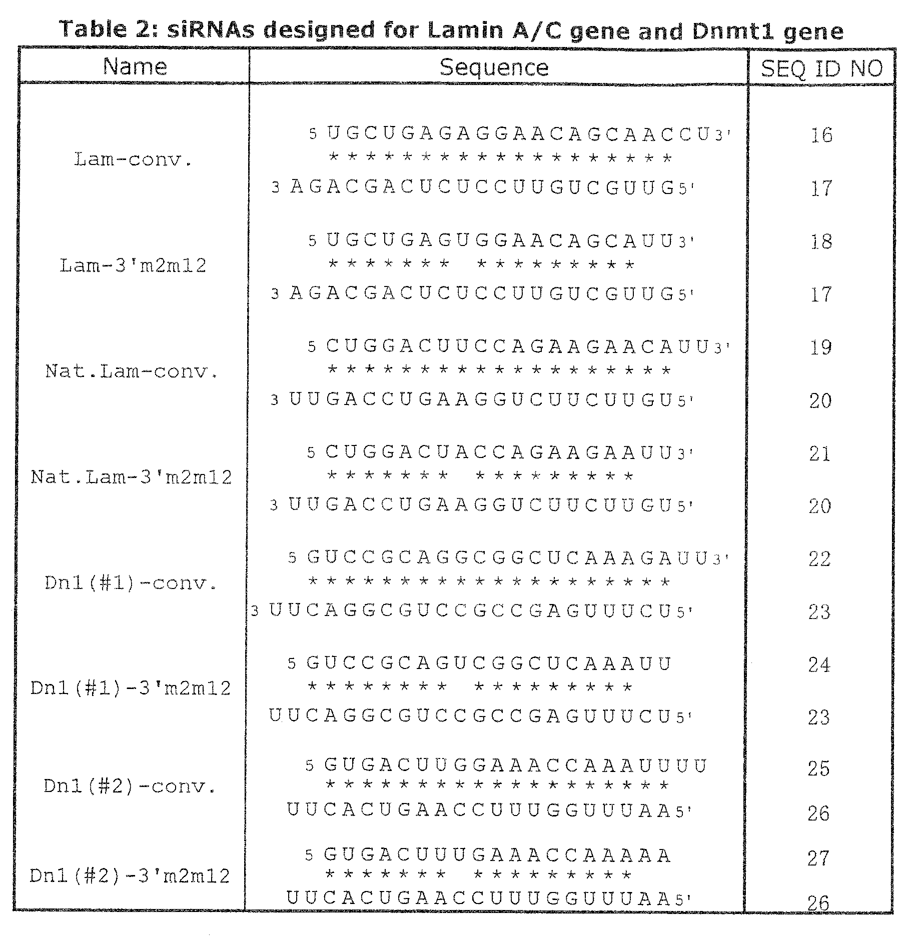

The abovementioned paired oligoribonucleotides were synthesized and 20 µM each of them were each mixed in an annealing buffer (30 mM HEPES pH 7.0, 100 mM potassium acetate, and 10 mM magnesium acetate), heat-denatured at 90°C for 3 minutes, and then annealed at 37°C for 24 hours. In this way, the individual siRNAs were obtained.

HeLa cells were grown at 37°C in Dulbecco's modified Eagle's medium (Sigma) supplemented with 10% fetal bovine serum (Life Technologies), 100 U/ml penicillin (Life Technologies) and 100 µg/ml streptomycin (Life Technologies) in a 5% CO₂ atmosphere.

Transfection of each siRNA (100 nM) into HeLa cells was carried out using a jetSI transfection reagent (Polyplus-transfection).

Total RNA was extracted 48 hours after transfection and subjected to cDNA synthesis by reverse transcriptase. The level of expression of the target gene was measured by real-time PCR using this cDNA as a template. A series of these expression level measurements were carried out using a SYBER Green PCR kit

### (Molecular Probe).

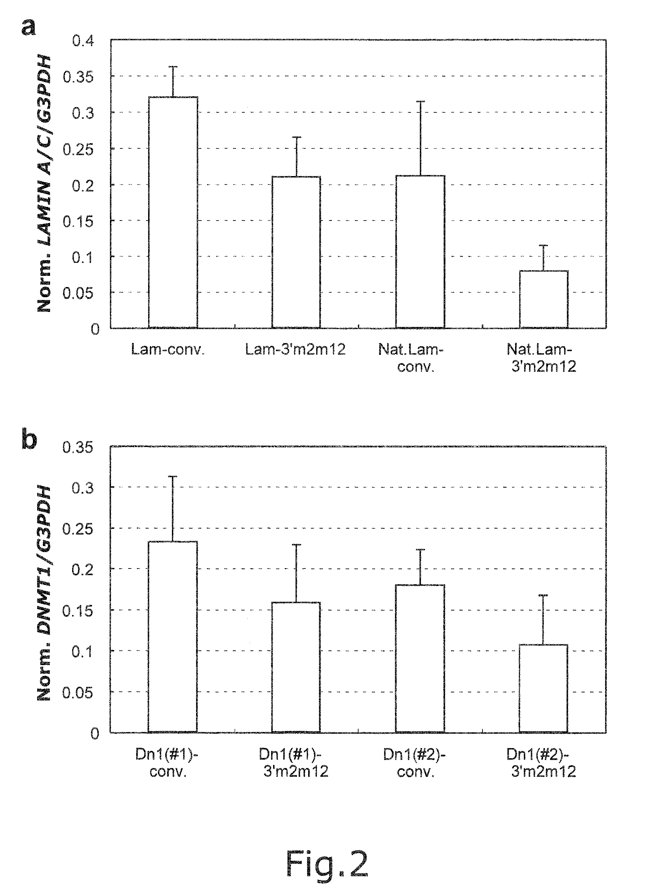

Fig. 2 shows the effect of the various siRNAs on suppressing the expression of the endogenous genes in HeLa cells. In Fig. 2, ratios of the expression level of target genes (Lamin A/C or Dnmt1 gene) to that of the control gene (G3PDH gene) are shown. These expression level ratios are normalized to the ratio obtained for a control sample using a double-stranded RNA (Mock, Qiagen) which triggers no gene expression suppression, setting the ratio to be 1.0. Data are averages of at least four independent experiments and error bars on the graph represent standard errors.

As shown in Fig. 1a and Fig. 2b, it is evident that the siRNAs carrying a mismatch in the nucleotide at position 12 from the 3'-end of the sense strand in addition to two nucleotide mismatches in the 3'-end of the sense strand demonstrated a higher level of gene expression-suppressing effect as compared to the conventional siRNAs.

### Example 3: Effect on RISC formation by mismatches in the 3'-end of the sense strand of siRNA molecule

When an siRNA is introduced into a cell, either its sense strand or antisense strand is incorporated into RISC. It is believed that the RISC thus formed cleaves mRNA with which the incorporated strand hybridizes. Therefore, it is assumed that the efficiency of the suppression of the target gene expression is different depending on which strand, i.e., the sense strand or antisense strand, of the siRNA is easily incorporated into RISC.

In this example, the following experiment was performed in order to examine whether the introduction of mismatches in the 3'-end of the sense strand of an siRNA molecule changes the selectivity for the strand to be incorporated into RISC.

First, two types of reporter plasmids were constructed in each of which a sequence corresponding to the sense strand or antisense strand of the target sequence "La21" of the *Photinus* luciferase gene was inserted into the 3' nontranslated region of the *Renilla* luciferase gene. As a control plasmid, a plasmid expressing the β-galactosidase gene was used. As siRNA molecules (dimers), La21.conv. (carrying no mismatch in the 3'-end of the sense strand) and La21-3'm2 (containing two nucleotide mismatches in the 3'-end of the sense strand) shown in Table 1 above were used.

HeLa cells were grown at 37°C in Dulbecco's modified Eagle's medium (Sigma) supplemented with 10% fetal bovine serum (Life Technologies), 100 U/ml penicillin (Life Technologies) and 100 µg/ml streptomycin (Life Technologies) in a 5% CO₂ atmosphere.

One of the abovementioned reporter plasmids, one of the abovementioned siRNA molecules, and the control plasmid were cotransfected into HeLa cells. Luciferase activity and β-galactosidase activity were measured 24 hours after cotransfection, and the value obtained by dividing the measured value for luciferase activity by the measured value for β-galactosidase activity was evaluated as a normalized reporter expression level.

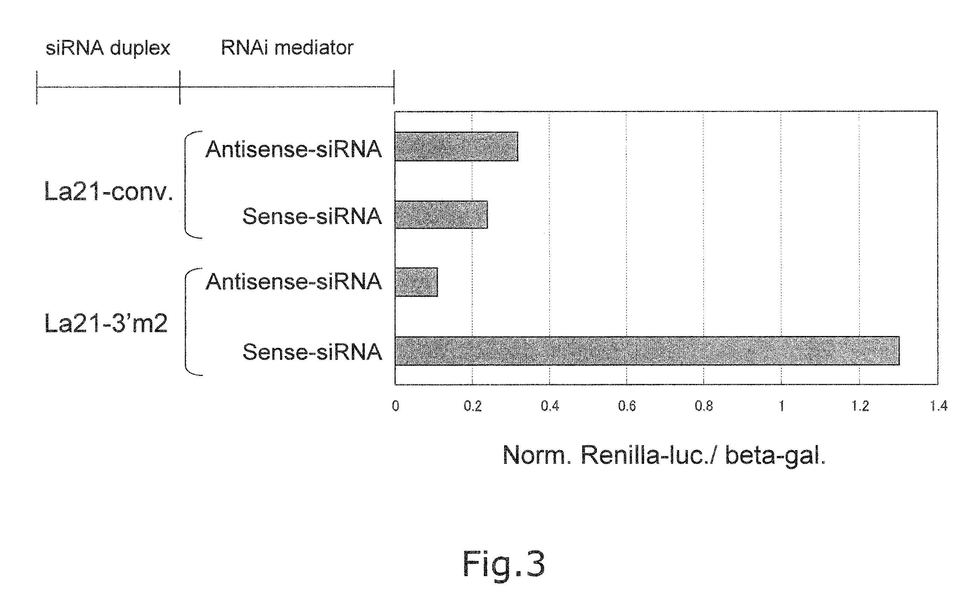

Fig. 3 shows bar graphs demonstrating the level of expression of the reporters from the two kinds of plasmids by the siRNA molecule (La21-3'm2) containing mismatches at the 3'-end of the sense strand or the siRNA molecule (La21-conv.) without mismatches. In Fig. 3, the graph "antisense-siRNA" demonstrates the expression level from the reporter plasmid in which the sense strand of the target sequence was incorporated into the 3' nontranslated region and thus this expression level was suppressed by RNAi in which the antisense strand of each siRNA molecule functioned as a mediator. On the other hand, the graph "sense-siRNA" demonstrates the expression level from the reporter plasmid in which the antisense strand of the target sequence was incorporated into the 3' nontranslated region and thus this expression level was suppressed by RNAi in which the sense strand of each siRNA molecule functioned as a mediator.

Fig. 3 demonstrates that when the conventional siRNA molecule containing no mismatch at the 3'-end of the sense strand (La21-conv.) was used, the sense strand and the antisense strand functioned as an RNAi mediator to almost the same extent. On the other hand, when the siRNA molecule containing mismatches at the 3'-end of the sense strand (La21-3'm2) was used, sense strand-mediated RNAi activity was hardly induced whereas antisense-mediated RNAi activity was markedly high. These results reveal that by introducing mismatches in the 3'-end of sense strand in the siRNA molecule, its antisense strand is preferentially incorporated into RISC as an RNAi mediator. This suggests that the siRNA molecule is incorporated into the RISC from the 5'-end of its antisense strand by mismatches at the 3'-end of the sense strand and as a result said antisense strand functions as an RNAI mediator.

## Claims

1. A double-stranded RNA molecule capable of suppressing the expression of a target gene in a cell by RNAi, which is designed such that one or more nucleotides in order from the 3'-end of the sense strand of double-stranded part in said RNA molecule are not co mplementary to the antisense strand,
wherein the sense strand of the double-stranded part has adequate number of nucleotides which are complementary to the antisense strand for enabling the hybridization of both strands in said cell.

2. The double-stranded RNA molecule according to claim 1, wherein the number of the nucleotides which are not complementary to the antisense strand in order from the 3'-end of the sense strand of the double-stranded part is 1 to 4.

3. The double-stranded RNA molecule according to claim 1, wherein the number of the nucleotides which are not complementary to the antisense strand in order from the 3'-end of the sense strand of the double-stranded part is 2.

4. The double-stranded RNA molecule according to claim 1, which is designed such that one additional nucleotide located at position 11-13 from the 3'-end of the sense strand of the double-stranded part is not complementary to the antisense strand.

5. The double-stranded RNA molecule according to claim 4, which is designed such that a nucleotide located at position 12 from the 3'-end of the sense strand of the double-stranded part is not complementary to the antisense strand.

6. The double-stranded RNA molecule according to claim 1, which is designed such that one additional nucleotide located at nucleotide position 1-3 in 5'- or 3'-direction from a site on the sense strand of the double-stranded part is not complementary to the antisense strand, the site corresponding to the cleavage site of the target gene transcription product by RISC.

7. The double-stranded RNA molecule according to claim 1, which is designed such that one additional nucleotide located at nucleotide position 1-3 in 5'-direction from the nucleotide in the center of the sense strand of the double-stranded part is not complementary to the antisense strand when the double-stranded part of the sense strand has an odd number of nudeotides, and that one additional nucleotide located at nucleotide position 1-3 in 5'-direction from the nucleotide at the 3'-side of the center of the sense strand of the double-stranded part is not complementary to the antisense strand when the double-stranded part of the sense strand has an even number of nucleotides.

8. The double-stranded RNA molecule according to claim 1, which is designed such that one additional nucleotide located at nucleotide position 2 in 5'-direction from the nucleotide in the center of the sense strand of the double-stranded part is not complementary to the antisense strand when the double-stranded part of the sense strand has an odd number of nucleotides, and that one additional nucleotide located at nucleotide position 2 in 5'-direction from the nucleotide at the 3'-side of the center of the sense strand of the double-stranded part is not complementary to the antisense strand when the double-stranded part of the sense strand has an even number of nucleotides.

9. The double-stranded RNA molecule according to claim 1, which does not induce double-stranded RNA-dependent protein kinase or 2',5'-oligoadenylate synthetase in a mammalian cell.

10. The double-stranded RNA molecule according to claim 9, which has a strand length of 29 or less nucleotides.

11. A double-stranded RNA molecule capable of suppressing the expression of a target gene in a cell by RNAi, which is designed such that one or more nucleotides in order from the 5'-end of the sense strand of double-stranded part in said RNA molecule are not complementary to the antisense strand,
wherein the sense strand of the double-stranded part has adequate number of nucleotides which are complementary to the antisense strand for enabling the hybridization of both strands in said cell.

12. The double-stranded RNA molecule according to claim 11, wherein the number of the nucleotides which are not complementary to the antisense strand in order from the 5'-end of the sense strand of the double-stranded part is 1 to 4.

13. The double-stranded RNA molecule according to claim 11, wherein the number of the nucleotides which are not complementary to the antisense strand in order from the 5'-end of the sense strand of the double-stranded part is 2.

14. The double-stranded RNA molecule according to claim 11, which is designed such that one or more additional nucleotides in order from the 3'-end of the sense strand of the double-stranded part are not complementary to the antisense strand.

15. The double-stranded RNA molecule according to claim 14, wherein the number of the nucleotides which are not complementary to the antisense strand in order from the 3'-end of the sense strand of the double-stranded part is 1 to 4.

16. The double-stranded RNA molecule according to claim 14, wherein the number of the nucleotides which are not complementary to the antisense strand in order from the 3'-end of the sense strand of the double-stranded part is 2.

17. The double-stranded RNA molecule according to claim 11, which is designed such that one additional nucleotide located at position 11-13 from the 3'-end of the sense strand of the double-stranded part is not complementary to the antisense strand.

18. The double-stranded RNA molecule according to claim 17, which is designed such that a nucleotide located at position 12 from the 3'-end of the sense strand of the double-stranded part is not complementary to the antisense strand.

19. The double-stranded RNA molecule according to claim 11, which is designed such that one additional nucleotide located at nucleotide position 1-3 in 5'- or 3'-direction from a site on the sense strand of the double-stranded part is not complementary to the antisense strand, the site corresponding to the cleavage site of the target gene transcription product by RISC.

20. The double-stranded RNA molecule according to claim 11, which is designed such that one additional nucleotide located at nucleotide position 1-3 in 5'-direction from the nucleotide in the center of the sense strand of the double-stranded part is not complementary to the antisense strand when the double-stranded part of the sense strand has an odd number of nucleotides, and that one additional nucleotide located at nucleotide position 1-3 in 5'-direction from the nucleotide at the 3'-side of the center of the sense strand of the double-stranded part is not complementary to the antisense strand when the double-stranded part of the sense strand has an even number of nucleotides.

21. The double-stranded RNA molecule according to claim 11, which is designed such that one additional nucleotide located at nucleotide position 2 in 5'-direction from the nucleotide in the center of the sense strand of the double-stranded part is not complementary to the antisense strand when the double-stranded part of the sense strand has an odd number of nucleotides, and that one additional nucleotide located at nucleotide position 2 in 5'-direction from the nucleotide at the 3'-side of the center of the sense strand of the double-stranded part is not complementary to the antisense strand when the double-stranded part of the sense strand has an even number of nucleotides.

22. The double-stranded RNA molecule according to claim 11, which does not induce double-stranded RNA-dependent protein kinase or 2',5'-oligoadenylate synthetase in a mammalian cell.

23. The double-stranded RNA molecule according to claim 22, which has a strand length of 29 or less nucleotides.

24. A method for suppressing the expression of a target gene in a cell, comprising a step of introducing the double-stranded RNA molecule according to any one of claims 1-23 into the cell.

25. The method according to claim 24, wherein the cell is a mammalian cell.

26. A vector comprising both of a DNA encoding the sense strand of the double-stranded RNA molecule according to any one of claims 1-23 and a DNA encoding the antisense strand of said RNA molecule.

27. A method for suppressing the expression of a target gene in a cell, comprising a step of introducing a combination of a vector containing a DNA encoding the sense strand of the double-stranded RNA molecule according to any one of claims 1-23 and a vector containing a DNA encoding the antisense strand of said RNA molecule, or a vector according to claim 26, into the cell.

28. The method according to claim 27, wherein the cell is a mammalian cell.

29. A double-stranded RNA molecule capable of suppressing the expression of a target gene in a cell by RNAi, which is modified such that said double-stranded RNA molecule is incorporated into an RNA-induced silencing complex from the side of 5'-end of the antisense strand.

30. A double-stranded RNA molecule capable of suppressing the expression of a target gene in a cell by RNAi, which is modified such that said double-stranded RNA molecule is incorporated into an RNA-induced silencing complex from the side of 5'-end of the sense strand.


SEQUENCE LISTING

<110> Japan Health Sciences Foundation

<120> Improved siRNA Molecule and Method of Suppressing Gene
Expression Using Thereof

<130> 66412/D24293 BC

<150> JP 2003-294504
<151> 2003-08-18

<150> JP 2003-427970
<151> 2003-12-24

<160> 30

<170> PatentIn Ver. 2.0

<210> 1
<211> 21
<212> RNA
<213> Artificial Sequence

<220>
<223> Description of Artificial Sequence:One Strand of
siRNA

<400> 1
ggaagacgcc aaaaacauau u 21

<210> 2
<211> 21
<212> RNA
<213> Artificial Sequence

<220>
<223> Description of Artificial Sequence:One Strand of
siRNA

<400> 2
uauguuuuug gcgucuuccu u 21

<210> 3
<211> 19
<212> RNA
<213> Artificial Sequence

<220>
<223> Description of Artificial Sequence:One Strand of
siRNA

<400> 3
ggaagacgcc aaaaacauu 19

<210> 4
<211> 19
<212> RNA
<213> Artificial Sequence

<220>
<223> Description of Artificial Sequence:One Strand of
siRNA

<400> 4
ggaagacgcc aaaaacaau 19

<210> 5
<211> 19
<212> RNA
<213> Artificial Sequence

<220>
<223> Description of Artificial Sequence:One Strand of
siRNA

<400> 5
ggaagacgcc aaaaacuau 19

<210> 6
<211> 19
<212> RNA
<213> Artificial Sequence

<220>
<223> Description of Artificial Sequence:One Strand of
siRNA

<400> 6
ggaagacgcc aaaaauuau 19

<210> 7
<211> 21
<212> RNA
<213> Artificial Sequence

<220>
<223> Description of Artificial Sequence:One Strand of
siRNA

<400> 7
uuaagacgcc aaaaacauau u 21

<210> 8
<211> 19
<212> RNA
<213> Artificial Sequence

<220>
<223> Description of Artificial Sequence:One Strand of
      siRNA

<400> 8
ggaagacucc aaaaacaau                                                    19

<210> 9
<211> 21
<212> RNA
<213> Artificial Sequence

<220>
<223> Description of Artificial Sequence:One Strand of
      siRNA

<400> 9
accgcuggag agcaacugcu u                                                 21

<210> 10
<211> 21
<212> RNA
<213> Artificial Sequence

<220>
<223> Description of Artificial Sequence:One Strand of
      siRNA

<400> 10
gcaguugcuc uccagcgguu u                                                 21

<210> 11
<211> 19
<212> RNA
<213> Artificial Sequence

<220>
<223> Description of Artificial Sequence:One Strand of
      siRNA

<400> 11
accgcuggag agcaacugu                                                    19

<210> 12
<211> 19
<212> RNA
<213> Artificial Sequence

<220>
<223> Description of Artificial Sequence:One Strand of
      siRNA

<400> 12
accgcuggag agcaacuuu                                                    19

<210> 13
<211> 19
<212> RNA
<213> Artificial Sequence

<220>
<223> Description of Artificial Sequence:One Strand of
      siRNA

<400> 13
accgcuggag agcaacauu                                                    19

<210> 14
<211> 19
<212> RNA
<213> Artificial Sequence

<220>
<223> Description of Artificial Sequence:One Strand of
siRNA

<400> 14
accgcuggag agcaauauu 19

<210> 15
<211> 19
<212> RNA
<213> Artificial Sequence

<220>
<223> Description of Artificial Sequence:One Strand of
siRNA

<400> 15
accgcuguag agcaacuuu 19

<210> 16
<211> 21
<212> RNA
<213> Artificial Sequence

<220>
<223> Description of Artificial Sequence:One Strand of
siRNA

<400> 16
ugcugagagg aacagcaacc u 21

<210> 17
<211> 21
<212> RNA
<213> Artificial Sequence

<220>
<223> Description of Artificial Sequence:One Strand of
siRNA

<400> 17
guugcuguuc cucucagcag a 21

<210> 18
<211> 19
<212> RNA
<213> Artificial Sequence

<220>
<223> Description of Artificial Sequence:One Strand of
siRNA

<400> 18
ugcugagugg aacagcauu 19

<210> 19
<211> 21
<212> RNA
<213> Artificial Sequence

<220>
<223> Description of Artificial Sequence:One Strand of
siRNA

<400> 19
cuggacuucc agaagaacau u 21

<210> 20
<211> 21
<212> RNA
<213> Artificial Sequence

<220>
<223> Description of Artificial Sequence:One Strand of
      siRNA

<400> 20
uguucuucug gaaguccagu u                                                21

<210> 21
<211> 19
<212> RNA
<213> Artificial Sequence

<220>
<223> Description of Artificial Sequence:One Strand of
      siRNA

<400> 21
cuggacuacc agaagaauu                                                   19

<210> 22
<211> 22
<212> RNA
<213> Artificial Sequence

<220>
<223> Description of Artificial Sequence:One Strand of
      siRNA

<400> 22
guccgcaggc ggcucaaaga uu                                               22

<210> 23
<211> 22
<212> RNA
<213> Artificial Sequence

<220>
<223> Description of Artificial Sequence:One Strand of
      siRNA

<400> 23
ucuuugagcc gccugcggac uu                                                22

<210> 24
<211> 20
<212> RNA
<213> Artificial Sequence

<220>
<223> Description of Artificial Sequence:One Strand of
      siRNA

<400> 24
guccgcaguc ggcucaaauu                                                   20

<210> 25
<211> 21
<212> RNA
<213> Artificial Sequence

<220>
<223> Description of Artificial Sequence:One Strand of
      siRNA

<400> 25
gugacuugga aaccaaauuu u                                                 21

<210> 26
<211> 21
<212> RNA
<213> Artificial Sequence

<220>
<223> Description of Artificial Sequence:One Strand of
siRNA

<400> 26
aauuugguuu ccaagucacu u 21

<210> 27
<211> 19
<212> RNA
<213> Artificial Sequence

<220>
<223> Description of Artificial Sequence:One Strand of
siRNA

<400> 27
gugacuuuga aaccaaaaa 19

<210> 28
<211> 19
<212> RNA
<213> Artificial Sequence

<220>
<223> Description of Artificial Sequence:One Strand of
siRNA

<400> 28
ggaagacgcc aaaaacaua 19

<210> 29
<211> 21
<212> RNA
<213> Artificial Sequence

<220>
<223> Description of Artificial Sequence:One Strand of
      siRNA

<400> 29
uacgcuggag agcaacugcu u                                                21

<210> 30
<211> 19
<212> RNA
<213> Artificial Sequence

<220>
<223> Description of Artificial Sequence:One Strand of
      siRNA

<400> 30
accgcuggag agcaacugc                                                   19